Europäisches Patentamt

European Patent Office

Office européen des brevets

(19)

(11) Veröffentlichungsnummer: **0 035 959**
**B1**

(12) # EUROPÄISCHE PATENTSCHRIFT

(45) Veröffentlichungstag der Patentschrift:
21.05.86

(51) Int. Cl.⁴: **A 61 N 1/05**

(21) Anmeldenummer: **81730022.1**

(22) Anmeldetag: **05.03.81**

(54) **Elektrode für einen künstlichen Herzschrittmacher.**

(30) Priorität: **06.03.80 DE 8006281 U**

(43) Veröffentlichungstag der Anmeldung:
**16.09.81 Patentblatt 81/37**

(45) Bekanntmachung des Hinweises auf die Patenterteilung:
**21.05.86 Patentblatt 86/21**

(84) Benannte Vertragsstaaten:
**AT CH DE FR GB IT LI NL SE**

(56) Entgegenhaltungen:
**EP - A - 0 015 229**
**FR - A - 2 297 641**
**US - A - 4 046 151**

**BIOMEDIZINISCHE TECHNIK, Band 24, no. 1-2, 1979,**
**Berlin, DE H.J. BISPING "Übersicht über verankerbare**
**transvenöse Schrittmachersonden", Seiten 16-27**

(73) Patentinhaber: **BIOTRONIK Mess- und Therapiegeräte**
**GmbH & Co Ingenieurbüro Berlin, Sieversufer 8,**
**D-1000 Berlin 47 (DE)**

(72) Erfinder: **Karr, Dieter E.Dipl.-Phys.,**
**Hans-Reyhing-Strasse 47, D-7250 Leonberg (DE)**

(74) Vertreter: **Christiansen, Henning, Dipl.-Ing.,**
**Dietrich-Schäfer-Weg 21, D-1000 Berlin 41 (DE)**

### Beschreibung

Die Erfindung betrifft eine Elektrodenanordnung für einen künstlichen Herzschrittmacher, welche am distalen Ende zwei zum Eingriff in das Körpergewebe dienende Befestigungshaken aus elektrisch leitendem Werkstoff aufweist, die mit tangential gerichteten zugespitzten Enden versehen sind, und welche einen in einer Stirnfläche endenden äußeren Mantel aus elektrisch isolierendem Material aufweist.

Eine derartige Elektrodenanordnung ist für die Ableitung fetaler Elektrokardiogramme bereits aus der US-PS 3 750 650 bekannt.

Im Gegensatz zu derartigen transvenös einzuführenden Elektrodenanordnungen, deren Befestigungsmittel durch ein vor dem endgültigen Fixieren der Elektrodenanordnung zu beseitigendes Schutzelement vor dem vorzeitigen Verhaken beim Einführen geschützt sind - oder mittels eines röhrenförmigen Katheders eingeführt werden -, liegt der erfindungsgemäßen Elektrodenanordnung die Aufgabe zugrunde, eine Gestaltung des Elektrodenkopfes anzugeben, welche es ermöglicht, die Elektrodenanordnung auch ohne zusätzliche Schutzmaßnahmen unter Vermeidung der Gefahr eines vorzeitigen Verhakens bzw. der Beschädigung von Herzklappen oder des Veneninneren zu ihrer Fixierungsposition voranzuschieben.

Diese Aufgabe wird für eine Elektrodenanordnung der obengenannten Art mit den im Kennzeichen des Anspruchs 1 angegebenen Maßnahmen erreicht.

Der Erfindung liegt das Bestreben zugrunde, den Elektrodenkopf allein durch seine Form derart auszubilden, daß der Befestigungshaken beim transvenösen Einführen nicht vorzeitig in das Gewebe eingreifen kann. Zum Eingreifen des Hakens in das Gewebe sollen dabei Kraftwechselwirkungen ausgenutzt werden, welche nur dann auftreten, wenn die Elektrodenanordnung ihre Fixierungsposition erreicht hat.

Durch die erfindungsgemäßen Maßnahmen werden Bereiche Erhebungen geschaffen, welche das Gewebe von dem zugespitzten Ende des Befestigungshakens abweisen, wenn sich die Ebene der Gewebeoberfläche in wesentlichen parallel oder in einem spitzen Winkel zur Elektrodenlängsrichtung erstreckt, wie es beim Voranschieben im Veneninneren der Fall ist. Hierbei ist es bei der erfindungsgemäßen Elektrodenanordnung nicht - wie bei den bekannten ungeschützten Elektrodenanordnungen - erforderlich, die Elektrodenanordnung beim Einführen ausschließlich entgegengesetzt rotierend zum Drehsinn beim Befestigen zu führen. Die Elektrodenanordnung kann vielmehr auch ohne Drehung vorangeschoben werden bzw. ein Eingriff in das Gewebe ist auch dann nicht zu befürchten, falls es zum weiteren Voranschieben oder zum Befreien aus einer Position, in der ein weiteres Voranschieben behindert ist, notwendig ist, die Elektrodenzuleitung kurzfristig ohne Druckausübung in Verhakungsrichtung zu drehen. Erst wenn die Elektrodenanordnung ihre Endposition im Ventrikel erreicht hat und die Herzwandung berührt, wobei das Gewebe in vollem Umfang an der Frontfläche der Elektrodenanordnung anliegt, bewirkt ein entsprechender vom distalen Ende her ausgeübter Druck, daß sich das Gewebe aufgrund seiner Formbarkeit auch in die die Erhebungen umgebenden konkaven Bereiche einschmiegt, in denen der Befestigungshaken zurückliegend befestigt ist.

Die der Spitzen der Befestigungshaken in der "Frontebene" benachbarten seitlichen Erhebungsbereiche erlauben also ein Eingreifen des Hakens in das Gewebe erst dann, wenn dieses bei Ausübung eines gewissen Druckes dicht an der Elektrodenfrontebene anliegt, wobei die Radien bzw. Abstände der Erhebungen von den Hakenenden derart bemessen sind, daß ein solcher Druck nur erzielbar ist, wenn der Elektrodenkopf sich mit seiner Frontebene im wesentlichen semkrecht auf einem Gewebebereich abstützt. Das Gewebe, in das die Befestigungshaken eingreifen sollen, gelangt infolge des ausgeübten Drucks in die Bereiche zwischen die Erhebungen umd damit in die Nähe der zugespitzten Enden der Befestigungshaken.

Auf diese Weise wird über die reine Trennung zwischen Vorschub- (beim Einführen) und Drehbewegung (zum Verhaken) mit einem aufzubringenden Mindestdruck beim Fixieren ein Kriterium geschaffen, welches die Befestigungshaken vor einem unbeabsichtigten Eindringen in das Gewebe schützt. Durch die in geringem Abstand von der Stirnfläche ders Elektrodenmantels tangential verlaufenden en Befestigungshaken ist die Gefahr einer Ventrikelperforation gering.

Ein vorteilhaftes Ausführungsbeispiel der erfindungsgemäßen Elektrodenanordnung (nachfolgend kurz als Elektrode bezeichnet ist in der Zeichnung dargestellt und wird nachfolgend näher beschrieben. Weitere bevorzugte Ausführungen sind in den Unteransprüchen angegeben. Es zeigen:

Figur 1 einen Längsschnitt des Ausführungsbeispiels, wobei die Zuleitung nur im Ansatz wiedergegeben ist, und

Figur 2 dasselbe Ausführungsbeispiel in der Draufsicht.

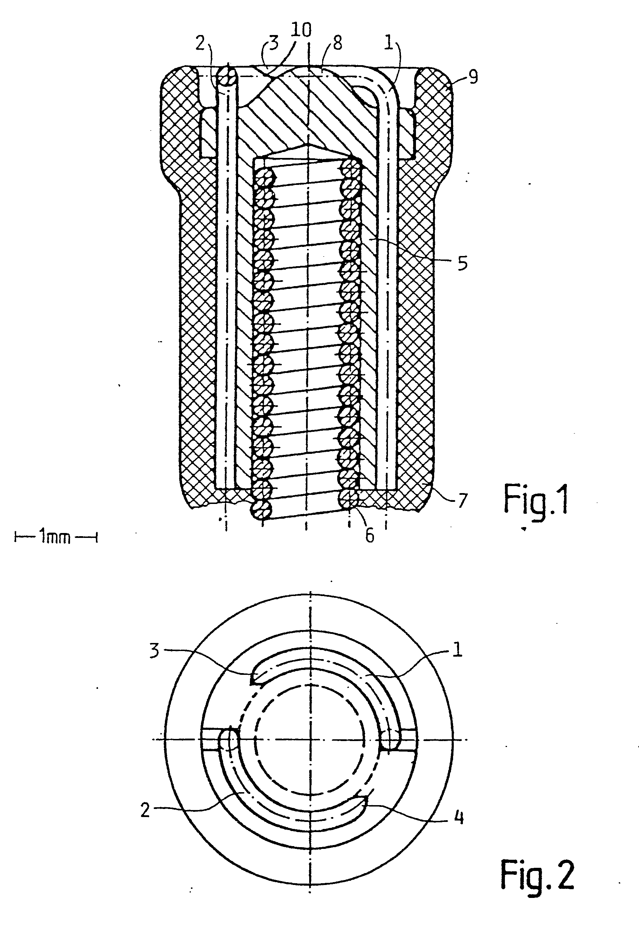

Bei der in Figur 1 dargestellten Ausführungsform sind zwei Befestigungshaken 1 und 2 mit zugespitzten Enden 3 und 4 (in Figur 2 erkennbar) an einem zylindrischen Elektrodenkopf 5 mittels Laser-Strahl punktförmig angeschweißt. Das Innere des Elektrodenkopfs 5 weist eine zylindrische Öffnung auf, in der die Zuleitungswendel 6, welche die elektrische Verbindung zum Schrittmacheraus- bzw. - eingang herstellt, endet. Als Werkstoff für die Metallteile dient eine körperverträgliche Kobaltlegierung. Die Zuleitungswendel 6 und der Elektrodenkopf 5 (mit Ausnahme der Frontfläche) ist mit einem Mantel 7 aus Silikonkautschuk umgeben, welcher eine elektrische Isolierung bildet. Die hakenförmigen Enden, die über die Elektrodenfrontfläche hinaus vorstehen, sind abgewinkelt und erstrecken sich in ihren Endbereichen parallel zur Frontfläche, wobei sie einen kreisbogenförmigen Verlauf konzentrisch zur Längsachse der Elektrode aufweisen.

Eine an der Frontfläche des Elektrodenkopfes vorhandene zentrale Erhebung 8 sowie eine einen vorspringenden Rand bildende ringförmige Erhebung 9 sind den frontalen Befestigungshaken 1 und 2 derart in einem Abstand benachbart angeordnet, daß sie eine nahezu geschlossene verrundete Oberflächenkontur bilden, welche Bezirke in der Umgebung der parallel zur Frontfläche verlaufenden Befestigungshaken der Enden ausspart.

Die zugespitzten Enden 3 und 4 sind damit beim Einführen optimal gegen ein unbeabsichtigtes Verhaken geschützt, da die Erhebungen 8 und 9 beim Voranschieben das Körpergewebe von den Enden abweisen. Die die Haken 1 und 2 umgebende Erhebung 9 besteht zweckmäßigerweise aus dem die Frontfläche der Elektrode überragenden Mantel 7. Die Erhebungen sind so verrundet und weisen in ihren bis in die Höhe der Befestigungshaken vorspringenden Bereichen ein solchen Abstand von deren zugespitzten Enden auf, daß sich das Körpergewebe nur bei entsprechender Druckausübung in axialer Richtung in die sich dazwischen ergebenden konkaven Vertiefungen einschmiegt. Die Verrundung des äußeren Randes erleichtert das Einführen der Elektrode durch die Vene.

Hat der Elektrodenkopf seinen zur Fixation vorgesehenen Ort erreicht, braucht zum Eingreifen in das Körpergewebe nun nur noch zusätzlich ein solcher Druck auf die in ihrem Stirnbereich im wesentlichen flach am Körpergewebe anliegende Elektrode über ihre Zuleitungswendel 6 ausgeübt werden, daß sich dieses in die zwischen der jeweiligen Spitze 3 bzw. 4 und den Erhebungen 8 bzw. 9 verbleibende Mulde hineinpreßt. Wird jetzt die Elektrodenzuleitung gedreht, so ist ein zuverlässiges Verhaken sichergestellt. Beim weiteren Drehen der Elektrode (in Figur 2 entgegengesetzt zum Uhrzeigersinn) dringen die Haken vollends ins Gewebe ein und halten den Elektrodenkopf fest.

Dieser Effekt wird noch dadurch unterstützt, daß die die Enden 3 und 4 in der Weise zugespitzt sind, daß jeweils eine Abschrägung 10 in dem der Frontfläche benachbarten Bereich angeordnet ist, die vorangeführte Seite jedoch unverkürzt ist, so daß im Frontbereich die Haken 1 und 2 einen nahezu geschlossenen Kreisring bilden (vgl. Figur 2). Damit wird das Körpergewebe beim reinen Voranschieben abgewiesen, beim Drehen in Verhakungsrichtung unter Ausübung eines gewissen Drucks aber sicher erfaßt.

Die erfindungsgemäßen Erhebungen können in verschiedener Weise zweckmäßig ausgestaltet sein, so weisen sie bevorzugterweise - in der Zeichnung nicht dargestellte - radial verlaufende Rippen oder Bereiche auf, in denen sie sich bezogen auf die Umfangsrichtung der Enden 3 oder 4 - diesen Haken unterschiedlich weit annähern, so daß sich beim Befestigen Gewebe in die hierdurch gebildeten Erweiterungen einfügt und durch die somit gebildeten, bezüglich der Eindringrichtung der Haken quer verlaufender Wülste eine Behinderung gegen unbeabsichtigtes Lösen der Elektrode gegeben ist.

**Patentansprüche**

1. Elektrodenanordnung für einen künstlichen Herzschrittmacher,
welche am distalen Ende zwei zum Eingriff in das Körpergewebe dienende Befestigungshaken aus elektrisch leitendem Werkstoff aufweist, die mit tangential gerichteten zugespitzten Enden versehen sind, und
welche einen in einer Stirnfläche endenden äußeren Mantel aus elektrisch isolierendem Material aufweist,
dadurch gekennzeichnet,
daß an der Frontfläche eines zentralen Elektrodenkopfes (5) und an der Stirnfläche des Mantels (7) Bereiche (8, 9) vorgesehen sind, welche mit den zugespitzten Enden der Befestigungshaken (1, 2) auf gleicher Höhe zu liegen kommen und in der so gebildeten Frontebene der Elektrodenanordnung solche Abstände von den zugespitzen Enden aufweisen, daß sich das Körpergewebe erst bei entsprechender axialer Druckausübung in die sich zwischen den zugespitzten Enden und den genannten Bereichen ergebenden zurückliegenden Teile einschmiegt.

2. Elektrodenanordnung nach Anspruch 1, dadurch gekennzeichnet, daß die Bereiche (8, 9) von den zugespitzten Enden der Befestigungshaken (1, 2) einen seitlichen Abstand von im wesentlichen einem Millimeter aufweisen.

3. Elektrodenanordnung nach einem der Ansprüche 1 oder 2, dadurch gekennzeichnet, daß die frontalen Abschnitte der Befestigungshaken (1, 2) einen kreisringförmigen Verlauf konzentrisch zur Längsachse des Elektrodenkopfes aufweisen, derart, daß sie einen nahezu geschlossenen Kreisring bilden.

4. Elektrodenanordnung nach Anspruch 3, dadurch gekennzeichnet, daß die Bereiche einen ringförmigen äußeren Rand (9) aufweisen.

5. Elektrodenanordnung nach einem der Ansprüche 3 oder 4, dadurch gekennzeichnet, daß die Bereiche eine zentrale Erhebung (8) aufweisen.

6. Elektrodenanordnung nach einem der vorangehenden Ansprüche, dadurch gekennzeichnet, daß die Erhebungen (8, 9) verrundete Konturen aufweisen.

7. Elektrodenanordnung nach Anspruch 4, dadurch gekennzeichnet, daß die kreisringförmige Erhebung (9) durch die die Frontfläche überragende, aus einem elektrisch isolierenden Werkstoff bestehende Ummantelung (7) des Elektrodenkopfes bzw. der Elektrodenzuleitung gebildet wird.

8. Elektrodenanordnung nach einem der vorangehenden Ansprüche, dadurch gekennzeichnet, daß die Befestigungshaken (1, 2) im Bereich ihrer zugespitzten Enden eine Abschrägung (10) an ihrer der Stirnfläche des Mantels zugewandten Seite aufweisen, welche sich in von den zugespitzten Enden (3) der Befestigungselemente (1) entfernender Richtung der Stirnfläche des Mantels annähert.

### Claims

1. An electrode arrangement for an artificial cardiac pacemaker which comprises, at the distal end, two attachment hooks of electrically conducting material which serve for engagement in the body tissue and which are provided with tangentially directed pointed ends, the electrode arrangement comprising an outer cover of electrically insulating material ending in an end face, characterised in that provided on the front face of a central electrode head (5) and on the end face of the cover (7) are regions (8, 9) which lie at the same level as the pointed ends of the attachment hooks (1, 2) and have such spacing from the pointed ends in the front plane, thus formed, of the electrode arrangement that the body tissue only fits snugly into the resulting parts recessed between the pointed ends and the said regions when appropriate axial pressure is exerted.

2. An electrode arrangement as claimed in Claim 1, characterised in that the regions (8, 9) have a lateral spacing from the pointed ends of the attachment hooks (1, 2) of substantially one millimetre.

3. An electrode arrangement as claimed in Claim 1 or 2, characterised in that the frontal portions of the attachment hooks (1, 2) have a circular course concentric with the longitudinal axis of the electrode head in such a manner that they form an almost closed circular ring.

4. An electrode arrangement as claimed in Claim 3, characterised in that the regions have an annular outer rim (9).

5. An electrode arrangement as claimed in Claim 3 or 4, characterised in that the regions have a central elevation (8).

6. An electrode arrangement as claimed in any one of the preceding Claims, characterised in that the elevations (8, 9) have rounded contours.

7. An electrode arrangement as claimed in Claim 4, characterised in that the circular elevation (9) is formed by the sheath (7) of the electrode head and/or of the electrode lead wire, which sheath projects beyond the front face and consists of an electrically insulating material.

8. An electrode arrangement as claimed in any one of the preceding Claims, characterised in that the attachment hooks (1, 2) comprise, in the region of their pointed ends, a bevel (10) at their side facing the end face of the cover, which approaches the end face of the cover in a direction extending away from the pointed ends (3) of the attachment elements (1).

### Revendications

1°) Dispositif à électrode pour un stimulateur cardiaque, présentant à l'extrémité eloignée deux crochets de fixation, en matériau électriquement conducteur, servant à s'engager dans le tissu du corps et munis d'extrémites epointées dirigées tangentiellement, et

présentant une enveloppe extérieure, en matériau électriquement isolant, se terminant dans une surface frontale, caractérisé

en ce qu'à la surface avant d'une tête centrale d'électrode (5) et à la surface frontale de l'enveloppe (7) sont prevues des zones (8, 9) qui viennent à la même hauteur que les extrémites épointées des crochets de fixation (1, 2) et présentent, dans le plan frontal, ainsi formé, du dispositif à électrode des distances, par rapport aux extrémités épointées, telles que le tissu du corps ne se replie dans les parties en retrait apparaissant entre les extrémités épointées et les zones mentionnées que sous l'action d'une pression axiale correspondante.

2°) Dispositif à électrode selon la revendication 1, caractérisé en ce que les zones (8, 9) présentent, par rapport aux extrémités épointées des crochets de fixation (1, 2), une distance latérale d'essentiellement un millimêtre.

3°) Dispositif à électrode selon les revendications 1 ou 2, caractérisé en ce que les tronçons frontaux des crochets de fixation (1, 2) présentent une allure en forme de couronne concentriquement à l'axe longitudinal de la tête d'électrode de façon à former un anneau circulaire presque fermé.

4°) Dispositif à électrode selon la revendication 3, caractérisé en ce que les zones présentent un bord extérieur annulaire (9).

5°) Dispositif à électrode selon l'une des revendications 3 ou 4, caractérisé en ce que les zones presentent une éminence centrale (8).

6°) Dispositif à électrode selon l'une des revendications précédentes, caractérisé en ce que les éminences (8, 9) présentent des profils arrondis.

7°) Dispositif à électrode selon la revendication 4, caractérisé en ce que l'éminence en forme d'anneau circulaire (9) est formée par l'enveloppe (7), constitué d'un matériau électriquement isolant et débordant au-delà de la surface frontale, de la tête d'électrode et du conducteur d'électrode.

8°) Dispositif à électode selon l'une des revendications précédentes, caractérisé en ce que les crochets de fixation(l, 2) présentent, dans la zone de leurs extrémités épointées, sur leur côté dirigé vers la surface frontale de l'enveloppe, un biseau qui s'approche de la surface frontale de l'enveloppe dans une direction qui va en s'éloignant des extrémités épointées (3) des éléments de fixation (1).

Fig.1

├─1mm─┤

Fig.2